# NEUE EUROPÄISCHE PATENTSCHRIFT

(11) **EP 2 620 426 B2**
(45) Veröffentlichungstag und Bekanntmachung des Hinweises auf die Entscheidung über den Einspruch: **21.02.2018**
(45) Hinweis auf die Patenterteilung: 19.03.2014
(21) Anmeldenummer: 12152872.3
(22) Anmeldetag: 27.01.2012
(51) Int. Cl.: C07C 273/04

(54) **Verfahren zur Herstellung von Harnstoff aus Abfällen, vorzugsweise Hausmüll, beliebiger Zusammensetzung**
Method for producing urea from waste, preferably domestic waste, of any composition
Procédé de fabrication d'urée à partir de déchets, de préférence des déchets ménagers de composition variée

(43) Veröffentlichungstag der Anmeldung: 31.07.2013
(73) Patentinhaber: Thermoselect AG, 9497 Triesenberg (LI)
(72) Erfinder: Kiss Günter Hans, 6600 Locarno (CH)
(74) Vertreter: Pfenning, Meinig & Partner mbB

(56) Entgegenhaltungen:
- WO-A1-02/090250
- CA-A1- 2 347 106
- US-A- 5 434 337
- US-A1- 2009 228 824
- US-B1- 6 455 011
- US-B1- 6 521 365
- YAMADA, S. ET AL.: "Thermoselect Waste Gasification and Reforming Process", JFE TECHNICAL REPORT, Bd. 3, 2004, Seiten 21-26, XP002677374,
- HIGMAN C. ET AL: 'Gasification', 2003, ELSEVIER, AMSTERDAM Seiten 231-239 - 293-295
- NIESSEN W.R.: 'Combustion and Incineration Processes', Bd. 3TH ED., 2002, MARCEL DEKKER, INC., NEW YORK

## Beschreibung

Die Erfindung betrifft ein Verfahren zur Herstellung von Harnstoff als stickstoffreichem Kunstdünger aus Abfällen beliebiger Zusammensetzung, vorzugsweise aus Hausmüll. Die organischen Müllbestandteile werden zunächst in einem Hochtemperaturreaktor mit Sauerstoff (O₂) vergast, der in einer kryogenen Luftzerlegungsanlage gewonnen worden ist. Es fällt ein Synthesegas an, das vor allem aus Kohlenmonoxid (CO), Wasserstoff (H₂) und Kohlendioxid (CO₂) besteht. Nachfolgend wird das im Synthesegas enthaltene Kohlenmonoxid (CO) mit Wasserdampf in Wasserstoff (H₂) und Kohlendioxid (CO₂) umgewandelt. Der Wasserstoff wird anschliessend getrennt und zusammen mit dem elementaren Stickstoff (N₂), der bei der kryogenen Luftzerlegung als Kuppelprodukt anfällt, zur Ammoniaksynthese eingesetzt. In der letzten Verfahrensstufe wird aus Ammoniak (NH₃) und der weiteren Synthesegaskomponente Kohlendioxid (CO₂) Harnstoff (CO(NH₂)₂) hergestellt.

Die Weltbevölkerung ist von 3 Mrd. in 1960 auf über 7 Mrd. in 2011 gestiegen. Auch in den nächsten Jahrzehnten muss mit einem überdurchschnittlichen Wachstum der Weltbevölkerung gerechnet werden, wobei der überwiegende Anteil des Bevölkerungswachstums derzeit in den Entwicklungsländern bzw. in den wenig entwickelten und ärmeren Staaten der Welt stattfindet.
Da die landwirtschaftlichen Nutzflächen begrenzt sind und da zusätzliche Nutzflächen nur in geringem Umfang geschaffen werden können, stellt die Ernährung der Weltbevölkerung ein immer grösseres Problem dar. Nur wenn es gelingt, die spezifischen Erträge der verfügbaren Nutzflächen signifikant zu erhöhen, besteht eine reelle Chance, die Weltbevölkerung auch zukünftig zu ernähren. Durch verstärkten Einsatz von Düngemitteln ist es möglich, die spezifischen Erträge der verfügbaren Nutzflächen zu erhöhen.

Harnstoff mit einem Stickstoffgehalt von 46,62 % ist weltweit der bedeutendste Stickstoffdünger. Harnstoff wird heute grosstechnisch aus Erdgas gewonnen. Dazu werden chemische Grossanlagen eingesetzt, die aus Erdgas, Luft und Wasser in den Prozessschritten Wasserstoffherstellung, Ammoniakherstellung, Harnstoffsynthese Harnstoff produzieren.

In 2009 wurden weltweit ca. 130 Mio. t Harnstoff produziert. In den letzten 3 Jahren ist der Harnstoffpreis um ca. 80 % auf 350 Euro/t gestiegen. Die steigenden Erdgaspreise und die steigende Nachfrage nach Harnstoff werden dazu führen, dass die Harnstoffpreise auch zukünftig überdurchschnittlich stark steigen werden.

Um die steigende Nachfrage nach Harnstoff als stickstoffhaltigem Kunstdünger befriedigen zu können, müssen Mittel und Wege gefunden werden, die jährliche Harnstoffproduktion signifikant zu erhöhen.

Mit dem Bevölkerungswachstum und dem zunehmenden Wohlstand der Bevölkerung ist auch das Müllaufkommen überproportional gestiegen, und die Suche nach einer umweltverträglichen Lösung des Müllproblems gewinnt eine immer grössere Bedeutung.

Mit thermischen Müllverwertungstechnologien wird versucht, das Müllproblem nachhaltig zu lösen. Unter diesen thermischen Verfahren nimmt das "Thermoselect-Verfahren" eine herausragende Stellung ein. Beim "Thermoselect-Verfahren" werden Abfälle unterschiedlichster Zusammensetzung und Konsistenz mit reinem Sauerstoff in einem Hochtemperaturreaktor bei einer Temperatur von bis zu 2.000° C vergast. Hierbei entsteht ein Synthesegas, das vor allem aus den Molekülen Wasserstoff (H₂), Kohlenmonoxid (CO) und Kohlendioxid (CO₂) besteht.

Der zur Vergasung der organischen Müllbestandteile eingesetzte Sauerstoff wird durch ein dem Stand der Technik entsprechendes kryogenes Luftzerlegungsverfahren gewonnen. Als Kuppelprodukt fällt hierbei elementarer Stickstoff an, der bisher in die Atmosphäre abgegeben wird. Das "Thermoselect-Verfahren" wird in der EP 0790291 B1 und der EP 0726307 B1 beschrieben.

Aufgabe der vorliegenden Erfindung ist es, Harnstoff kostengünstig aus den Produkten und Kuppelprodukten zu kostengünstigen Preisen herzustellen, die bei der Vergasung von Abfällen nach dem "Thermoselect-Verfahren" anfallen, ohne dass hierbei gasförmige Emissionen, wie z.B. CO₂, die Umwelt belasten.

Die vorstehende Aufgabe wird durch das erfindungsgemässe Verfahren gelöst, wie es im Patentanspruch definiert ist.

Das erfindungsgemässe Verfahren besteht aus folgenden Prozessstufen:
In der ersten Stufe wird durch eine Hochtemperaturvergasung der Abfälle mit reinem Sauerstoff nach dem "Thermoselect-Verfahren" Synthesegas, bestehend vor allem aus Kohlenmonoxid (CO), Kohlendioxid (CO₂) und Wasserstoff (H₂), erzeugt. Wesentlich ist hierbei, dass der benötigte Sauerstoff, nach dem kryogenen Luftzerlegungsverfahren gewonnen wird. Hierbei fällt als Kuppelprodukt elementarer Stickstoff an. Die Verweilzeit des Synthesegases im Reaktor beträgt hierbei 1,0 bis 5,0 Sekunden.

Anschließend erfolgt eine Verdichtung des bei der Hochtemperaturbehandlung gebildeten Synthesegases auf 10 bis 80 bar.

In der zweiten Stufe wird das im Synthesegas enthaltene Kohlenmonoxid (CO) mittels Wasserdampf (H₂O) in Kohlendioxid (CO₂) und Wasserstoff (H₂) umgewandelt. Bei diesem Verfahrensschritt wird bevorzugt der Wasserdampf eingesetzt, der bei der Vergasung des Abfalls im Hochtemperaturreaktor anfällt.

Nach Abtrennung des Wasserstoffs (H₂) vom Kohlendioxid (CO₂) wird der Wasserstoff (H₂) mit dem Stickstoff (N₂), der als Kuppelprodukt bei der Luftzerlegung anfällt, zu Ammoniak (NH₃) umgesetzt.

Im letzten Schritt wird aus Ammoniak (NH₃) und Kohlendioxid (CO₂) aus dem Synthesegas Harnstoff (CO(NH₂)₂) gewonnen.

Die wesentlichen Vorteile des erfindungsgemässen Verfahrens bestehen darin, dass
- Abfälle unterschiedlichster Zusammensetzung durch Vergasung mit Sauerstoff nach dem "Thermoselect-Verfahren" ohne Belastung der Umwelt vollständig in nutzbare Produkte transformiert werden, während bei allen anderen bekannten thermischen Verfahren die Umwelt durch zu deponierende hochtoxische Reststoffe und durch gasförmige Emissionen, wie z.B. CO₂, belastet wird, dass
- der elementare Stickstoff, der als Kuppelprodukt bei der Luftzerlegung anfällt, zusammen mit dem im geshifteten Synthesegas enthaltenen Wasserstoff zur Ammoniaksynthese genutzt werden, dass
- der als Zwischenprodukt gewonnene Ammoniak zusammen mit dem im geshifteten Synthesegas enthaltenen Kohlendioxid zur Harnstoffproduktion eingesetzt wird und dass
- bei dem erfindungsgemässen Verfahren keine gasförmigen Emissionen, wie z.B. CO₂, die Umwelt belasten. Sollte mehr CO₂ im geshifteten Synthesegas enthalten sein als zur Ammoniaksynthese benötigt wird, kann Ammoniak zugekauft werden, um auch das überschüssige CO₂ zur Ammoniaksynthese zu nutzen.

Bei Einsatz des hier vorgeschlagenen Verfahrens kann z.B. Künstdünger aus Harnstoff gewonnen werden, wobei pro Tonne Müll ca. 600 kg stickstoffreicher Kunstdünger entstehen. Der aktuelle Marktpreis dieses Düngers beträgt ca. 350 Euro/Tonne.

Mit einem spezifischen Überschuss von ca. 200 Euro/Tonne, der durch den Verkauf des stickstoffreichen Kunstdüngers erzielt werden kann, können alle Kosten des hier beschriebenen Verfahrens, einschliesslich aller Investitionsaufwendungen problemlos gedeckt werden. Unabhängig von der Höhe der Gebühren, die für die Entsorgung der Abfälle erzielt werden, ist eine überdurchschnittliche Verzinsung des eingesetzten Kapitals gewährleistet.

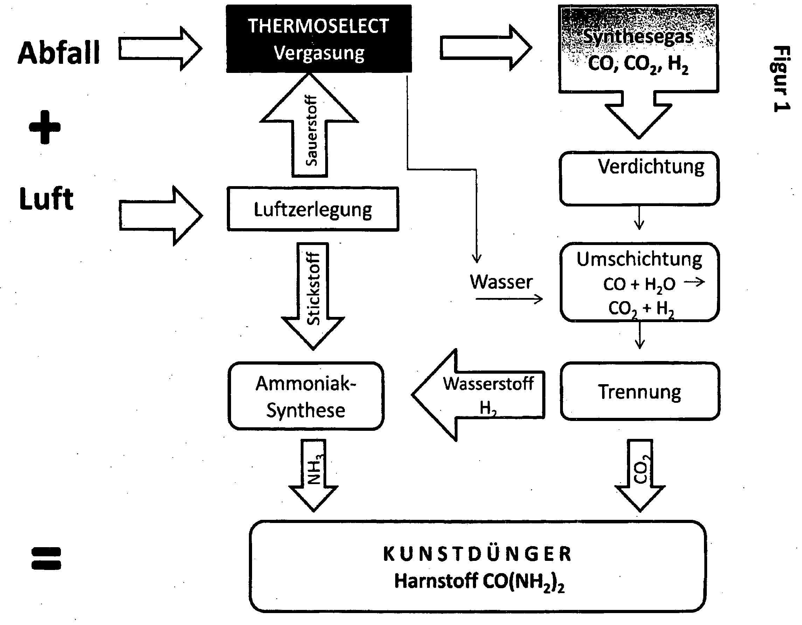

Die Erfindung wird nachfolgend anhand eines allgemeinen Verfahrensschemas (Figur 1) näher erläutert.

Zur Bildung des Synthesegases wird dabei der Abfall beliebiger Zusammensetzung bei einer Temperatur von mindestens 1000 °C bis 2000 °C, bevorzugt bei ca. 1500 °C, in einem Hochtemperaturreaktor mit O₂ vergast. Die Verweilzeit des Synthesegases im Reaktor beträgt 1,0 Sekunden bis 5,0 Sekunden, bevorzugt ca. 2,0 Sekunden. Um eine Rückbildung der Synthesegasbestandteile in toxische Produkte zu verhindern, wird das Synthesegas einer Schockkühlung (Quench) unterzogen.

Im nächsten Schritt wird das Synthesegas, das aus der vorstehend beschriebenen Hochtemperaturbehandlung gebildet wird, zunächst vorzugsweise auf 10 bis 80 bar, bevorzugt auf ca. 50 bar verdichtet.

Um das Synthesegas möglichst effizient für die Gewinnung von stickstoffreichem Kunstdünger zu nutzen, sollte der Wasserstoffanteil im Synthesegas möglichst hoch sein. Mit Hilfe der sogenannten, dem Stand der Technik entsprechenden, Shiftreaktion wird vorzugsweise in einem gekühlten Reaktor mit Katalysatorfestbett, durch Dosierung von Wasserdampf die Reaktion, gemäß der Formel CO + H₂0 = CO₂ + H₂, durchgeführt. Da durch die exotherme Reaktion Wärme entsteht, wird der Reaktor mit Wasser auf die für den Katalysator optimale Temperatur von 300 °C gekühlt.

Das geshiftete Synthesegas, das vorzugsweise aus Wasserstoff und Kohlendioxid besteht, wird in einer nächsten Verfahrensstufe - die ebenfalls dem Stand der Technik entspricht - gestrippt, indem der Wasserstoff abgetrennt wird. Es verbleibt ein Restgas, das fast ausschließlich aus Kohlendioxid besteht.

Der Wasserstoff wird zusammen mit dem Stickstoff aus der Luftzerlegungsanlage in einem Synthesereaktor katalytisch zur Gewinnung von Ammoniak eingesetzt.

In der nächsten Verfahrensstufe erfolgt die Harnstoffsynthese, indem Ammoniak und Kohlendioxid aus dem Restgas katalytisch zur Gewinnung von Harnstoff als stickstoffreichem Kunstdünger eingesetzt werden. Sollte die CO₂-Menge größer sein als der CO₂-Bedarf, der zur Harnstoffsynthese notwendig ist, so kann NH₃ zugekauft werden, um den CO₂-Überschuss zur Harnstoffsynthese einzusetzen.

Der Vorteil des hier beschriebenen Verfahrens besteht vor allem darin, dass die Müllverwertung zu keinen gasförmigen Emissionen führt. Alle Stoffe, die anfallen, sind industriell nutzbar. Zur Gewinnung von stickstoffreichem Kunstdünger werden ausschließlich die aus der Vergasung organischer Müllbestandteile nach dem "Thermoselect-Verfahren" gewonnenen Stoffe, wie Wasserstoff und Kohlendioxid, eingesetzt. Der zur Ammoniaksynthese notwendige elementare Stickstoff fällt in der Luftzerlegungsanlage als Kuppelprodukt an.

In einer Abwandlung des Verfahrens kann auch die für das Verfahren benötigte Energie aus regenerativen Energien z.B. der Fotovoltaik gewonnen werden.

## Patentansprüche

1. Verfahren zur umweltschonenden Herstellung von Harnstoff (CO(NH₂)₂)
**dadurch gekennzeichnet,**
**dass** Harnstoff aus Abfällen beliebiger Zusammensetzung hergestellt wird, umfassend die folgenden Schritte:
a) Erzeugung eines Synthesegases aus CO, CO₂ und H₂ durch Hochtemperaturbehandlung von Abfall in einem Reaktor mit O₂, der mittels einer kryogenen Luftzerlegungsverfahren erzeugt wird, wobei die Verweilzeit des Synthesegases im Reaktor 1,0 bis 5,0 Sekunden beträgt,
b) Verdichtung des bei der Hochtemperaturbehandlung gebildeten Synthesegases auf 10 bis 80 bar,
c) Umwandlung des im Synthesegas enthaltenen CO mit H₂O in CO₂ + H₂ (Shifting) und Abtrennen des CO₂ vom H₂,
d) Umsetzung des aus Schritt c) erhaltenen H₂ mit N₂, der aus der kryogenen Luftzerlegung aus Schritt a) stammt, zur Bildung von Ammoniak (NH₃) und
e) Umsetzung des NH₃ aus Schritt d) mit CO₂ aus Schritt c) zur Bildung von Harnstoff (CO(NH₂)₂)

## Claims

1. A method for the environmentally friendly manufacture of urea (CO(NH₂)₂), **characterized in that** urea is manufactured from waste of any composition, comprising the following steps:
a) generation of a synthesis gas from CO, CO₂ and H₂ by high-temperature treatment of waste in a reactor with O₂ which is produced by means of a cryogenic air separation process, wherein the residence time of the synthesis gas in the reactor is 1.0 to 5.0 seconds;
b) compression of the synthesis gas formed in the high-temperature treatment to 10 to 80 bar,
c) conversion of the CO contained in the synthesis gas using H₂O into CO₂ + H₂ (shifting) and separation of the CO₂ from the H₂;
d) conversion of the H₂ obtained from step c) using N₂ which originates from the cryogenic air separation from step a) for forming ammonia (NH₃); and
e) conversion of the NH₃ from step d) using CO₂ from step c) for forming urea (CO(NH₂)₂).

## Revendications

1. Procédé de préparation écologique d'urée (CO(NH₂)₂), **caractérisé en ce que** l'urée est préparée à partir de déchets de composition quelconque, comprenant les étapes suivantes:
a) production d'un gaz de synthèse à partir de CO, de CO₂ et d'H₂ par un traitement à haute température de déchets dans un réacteur avec de l'O₂, qui est produit par un procédé de décomposition cryogénique de l'air, dans lequel le temps de séjour du gaz de synthèse dans le réacteur est de 1,0 à 5,0 secondes,
b) compression du gaz de synthèse formé dans le traitement à haute température jusqu'à 10 à 80 bars,
c) conversion du CO contenu dans le gaz de synthèse avec de l'H₂O en CO₂ + H₂ (déplacement) et séparation du CO₂ de l'H₂,
d) réaction du H₂ obtenu à l'étape c) avec du N₂ qui provient de la décomposition cryogénique de l'air à l'étape a) pour former de l'ammoniac (NH₃), et
e) réaction du NH₃ à l'étape d) avec le CO₂ à l'étape c) pour former de l'urée (CO(NH₂)₂).
